# EUROPEAN PATENT SPECIFICATION

(11) **EP 3 516 398 B1**
(45) Date of publication and mention of the grant of the patent: **04.11.2020**
(21) Application number: 17771734.5
(22) Date of filing: 19.09.2017
(51) Int. Cl.: G01N 33/68, G01N 33/74

(54) **IN VITRO METHOD FOR DIAGNOSING AT EARLY STAGE INTESTINAL ISCHEMIA**
IN-VITRO-VERFAHREN ZUR DIAGNOSE EINES FRÜHSTADIUMS VON DARMISCHÄMIE
PROCÉDÉ IN VITRO POUR DIAGNOSTIQUER UNE ISCHÉMIE INTESTINALE EN PREMIER DÉVELOPPEMENT

(30) Priority: 20.09.2016 EP 16306199; 28.03.2017 WO PCT/EP2017/057292
(43) Date of publication of application: 31.07.2019
(73) Proprietor: Université de Bourgogne, 21078 Dijon (FR); INSERM (Institut National de la Santé et de la Recherche Médicale), 75013 Paris (FR); Institut National Supérieur des Sciences Agronomiques, de l'Alimentation et de l'Environnement, 21000 Dijon (FR)
(72) Inventor: GROBER, Jacques, 21310 Belleneuve (FR); LEBRUN, Lorène, 84210 PERNES LES FONTAINE (FR)
(74) Representative: IPAZ
(86) International application number: PCT/EP2017/073584
(87) International publication number: WO 2018/054881

(56) References cited:
- WO-A2-2007/079301
- US-A1- 2001 027 180
- POWELL ALEXIS ET AL: "Plasma biomarkers for early diagnosis of acute intestinal ischemia", SEMINARS IN VASCULAR SURGERY, vol. 27, no. 3, 29 April 2014 (2014-04-29) , pages 170-175, XP029163626, ISSN: 0895-7967, DOI: 10.1053/J.SEMVASCSURG.2015.01.008
- SHI HUI ET AL: "The role of serum intestinal fatty acid binding protein levels and D-lactate levels in the diagnosis of acute intestinal ischemia.", CLINICS AND RESEARCH IN HEPATOLOGY AND GASTROENTEROLOGY JUN 2015, vol. 39, no. 3, June 2015 (2015-06), pages 373-378, XP002766326, ISSN: 2210-741X
- DATABASE MEDLINE [Online] US NATIONAL LIBRARY OF MEDICINE (NLM), BETHESDA, MD, US; 2015, SIT M ET AL: "Serum omentin levels predicts mesenteric ischemia.", XP002766327, Database accession no. NLM25869566
- DRUCKER D J: "Gut adaptation and the glucagon-like peptides", GUT, vol. 50, no. 3, March 2002 (2002-03), pages 428-435, XP002766328, ISSN: 0017-5749

## Description

The present invention concerns an *in vitro* method for diagnosing at early stage of intestinal ischemia.

Acute mesenteric ischemia (AMI) is a severe medical emergency caused by a sudden decrease of blood flow through the mesenteric vessels, as a result of some diseases, trauma, shock, surgery, or organ transplantation. Mesenteric ischemia leads to cellular dysfunction and eventual gangrene of the bowel wall. AMI may be classified as either arterial or venous. Arterial AMI may be also classified as a nonocclusive mesenteric ischemia (NOMI) or an occlusive mesenteric arterial ischemia (OMAI). In large part because of the association with atherosclerosis, AMI is commonly considered a disease of the older population, with the typical age of onset being older than 60 years (Cardin et al., Aging Clin Exp Res. 2012 Jun. 24 (3 suppl): 43-46).

If mesenteric ischemia itself is deleterious for an organism, its treatment which consists in a reperfusion, could be worse. The bowel is one of the organs the most sensible to ischemia-reperfusion (Yamamoto et al., J. Surg. res., 2001, 99: 134-141). Intestinal mucosa can be rapidly impacted by a hypoperfusion (lack of oxygen). However, paradoxically, restoration of blood flow after ischemia (reperfusion) of ischemically damaged intestinal tissue may further aggravate tissue damage rather than decrease tissue damage as it abruptly reintroduces oxygen that stimulates the production of free radicals that promote or accelerate necrosis (Guan et al., Am J Physiol Gastrointest Liver Physiol 2009, 297: G187-G196). Ischemia followed by reperfusion (IR) can rapidly trigger the production of inflammation mediators. This local inflammation can be quickly generalized at the systemic level. The passing of pro-inflammatory bacterial endotoxins through intestinal barrier can result in systemic inflammation response syndrome (SIRS) state and dysfunction of other organs. Besides, intestinal ischemia-reperfusion is considered as a motor of multiple organ failure (MOF) state (Harward et al., J. Vasc. Surg. 1993, 18: 459-469).

Despite the last medical advances, the high mortality of this pathology has not changed since the 1940s: it concerns about 60% to 80% of patients suffering from the said disease (Schoots et al., Br. J. Surg. 2004 Jan. 91(1): 17-27). Once bowel wall infarction has occurred, mortality may be as high as 90%. Unfortunately, even with a good treatment, there is still 50 to 80% of the patients that die.

This high mortality is partially due to the difficulty to diagnose AMI at early stage. Before the appearance of warning signs of peritonitis, symptoms of AMI are initially nonspecific. Until now, there is no efficient biological marker which enables to diagnose AMI at early stage. Conventional clinical used markers are serum lactate level and white blood cell number. These markers have neither satisfactory specificity, nor sensibility and cannot diagnose AMI at early stage. Other existing diagnostic tests are angiography or tomodensitometry. They present many disadvantages, since they are invasive and could generate medical complications, such as those at the renal level (Glenister et Corker, ANSZ J. Surg. 2004, 74: 260-265).

In the last few years, a great clinical and preclinical effort has been done for seeking biomarkers which could predict gastro-intestinal damage before it is generated to systematic level. D-lactate (Demir et al., Dig. Surg., 2012, 29: 226-235), intestinal fatty acid binding protein "I-FABP" (Cronk et al., Curr. Surg. 2006, 63: 322-325), α-glutathione S-transferase (Khurana et al., 2002, J. Pediatr. Surg., 2002, 37: 1543-1548), albumin (Dundar et al., Acad. Emerg. Med. 2010, 17: 1233-1238) and D-dimer (Chiu et al., Am. J. Emerg. Med. 2009, 27: 975-979) are studied as potential biomarker for AMI diagnostic. I-FABP is particularly promising, since the concentration of plasma I-FABP in mouse is increased 30 minutes post ischemia followed by 2 hours of reperfusion, which enables to predict intestinal ischemia prior to the appearance of pathological evidence (Khadaroo et al., PloS One, 2014, 9: e115242).

Despite these promising results, I-FABP can detect ischemia only 30 min post ischemia. As such, it remains necessary to develop new biomarkers that have the capacity to diagnose AMI more rapidly and at more early stages.

The Inventors have surprisingly found in a mouse model of intestinal ischemia/reperfusion, that when a mouse suffers from intestinal ischemia, an increase of circulating glucagon-like peptide 1 (GLP-1) and glucagon-like peptide 2 (GLP-2) in the plasma level of the said mouse can be observed after only from 10 to 15 minutes of ischemia followed by 15 minutes of reperfusion. In another word, GLP-1 and GLP-2 are predictive at a very early stage of an intestinal ischemia in mice.

These experimental results suggest that GLP-1 and GLP-2 can also be predictive at a very early stage of an intestinal ischemia in human or other non-human mammals, particularly in patient suspected of suffering from intestinal ischemia.

Glucagon-like peptide 1 and glucagon-like peptide 2 are both created by specific post-translational proteolytic cleavage of proglucagon in intestinal endocrine L cells. GLP-1 and GLP-2 are co-secreted upon nutrient ingestion.

The human circulating GLP-1 protein is mainly secreted by intestinal L-cells predominantly localized in the distal small intestine and colon. The GLP-1 forms present in plasma can be: GLP-1-(7-37), GLP-1-(7-36)NH₂ and their degradation forms such as GLP-1-(9-36), GLP-1-(28-36).

The human circulating GLP-2 protein is a 33 amino acid peptide. In plasma, it exists the bioactive form GLP-2-(1-33) and its degradation product GLP-2-(3-33).

Therefore, the first aspect of the invention relates to an *in vitro* diagnostic method for diagnosing at an early stage an intestinal ischemia in a patient suspected of suffering from intestinal ischemia, which comprises:
(i) determining in a biological sample from said patient the circulating glucagon-like peptide 2 (GLP-2) level and/or the level of circulating glucagon-like peptide 1 (GLP-1) level,
(ii) comparing said level with the circulating glucagon-like peptide 2 level and/or the circulating glucagon-like peptide 1 level in a reference sample ,
wherein the increase of said level in the patient is indicative of said patient suffering from intestinal ischemia.

"A patient suspected of suffering from intestinal ischemia" refers to a patient who suffers from one of the diseases which are generally known as causes of intestinal ischemia or has predisposing conditions, such as a patient suffering from a cardiac emboli, emboli from fragments of proximal aortic thrombus, atheromatous plaque dislodged by arterial catheterization or surgery, an atherosclerotic vascular disease, aortic aneurysm, aortic dissection, arteritis, decreased cardiac output from myocardial infarction or congestive heart failure, dehydration from any cause, hypotension from congestive heart failure, myocardial infarction, sepsis, aortic insufficiency, severe liver or renal disease, or recent major cardiac or abdominal surgery, or a patient consuming vasopressors, ergotamines, cocaine, or digitalis, or a patient having hypercoagulability from protein C and S deficiency, antithrombin III deficiency, dysfibrinogenemia, abnormal plasminogen, polycythemia vera, thrombocytosis, sickle cell disease, factor V Leiden mutation, pregnancy, and oral contraceptive use, tumor causing venous compression or hypercoagulability, intra-abdominal infections, such as appendicitis, diverticulitis, or abscess, venous congestion from cirrhosis, venous trauma from accidents or surgery, especially portacaval surgery, increased intra-abdominal pressure from pneumoperitoneum during laparoscopic surgery, pancreatitis, decompression sickness.

The term "at early stage" refers to a period as short as 10 minutes of ischemia followed by 15 minutes of reperfusion.

The term "biological sample" refers to any biological sample which can be obtained from a patient, in particular a sample of body fluids, such as urine or blood. In a preferred embodiment of the present invention, the biological sample is a blood plasma sample.

A reference sample used in the *in vitro* method of the present invention is a biological sample obtained from a healthy subject.

The term "circulating glucagon-like peptide 1" refers to GLP-1 present in human plasma in form of GLP-1-(7-37), GLP-1-(7-36)NH₂ and GLP-1-(9-36).

The term "circulating glucagon-like peptide 2" refers to GLP-2 present in human plasma in form of GLP-2-(1-33) and GLP-2-(3-33).

When it is the level of GLP-1 that is measured in a biological sample from a patient, the step of comparison is carried out between said measured GLP-1 and the level of GLP-1 in a reference sample.

When it is the level of GLP-2 that is measured in a biological sample from a patient, the step of comparison is carried out between said measured GLP-2 and the level of GLP-2 in a reference sample.

When both the levels of GLP-1 and of GLP-2 are measured in a biological sample from a patient, the step of comparison is carried out between said measured GLP-1 level and GLP-2 level and those in a reference sample.

In an embodiment, the method of the present invention comprises:
(i) determining in a biological sample from said patient the level of circulating glucagon-like peptide 1 (GLP-1) level,
(ii) comparing said level with the circulating glucagon-like peptide 1 level in a reference sample ,
wherein the increase of said level in the patient is indicative of said patient suffering from intestinal ischemia.

According to the present invention, when GLP-1 level in a biological sample of a patient suspected of suffering from intestinal ischemia is at least 50%, 60%, 70%, 80%, 90%, 100%, 150%, 200%, 250%, or 300% higher than GLP-1 level measured at the same time in reference sample, it is considered that there is an increase of GLP-1 level in said patient and there is very high probability that said patient is suffering from an intestinal ischemia.

In another embodiment, the method of the present invention comprises:
(i) determining in a biological sample from said patient the level of circulating glucagon-like peptide 2 (GLP-2) level,
(ii) comparing said level with the circulating glucagon-like peptide 2 level in a reference sample ,
wherein the increase of said level in the patient is indicative of said patient suffering from intestinal ischemia.

When GLP-2 level in a biological sample of a patient suspected of suffering from intestinal ischemia is at least 50%, 60%, 70%, 80%, 90%, 100%, 150%, 200%, 250%, or 300% higher than GLP-2 level measured at the same time in reference sample, it is considered that there is an increase of GLP-2 level in said patient and there is very high probability that said patient is suffering from an intestinal ischemia.

An "intestinal ischemia" is referred to a variety of disorders that occur when blood flow in the gastrointestinal tract is insufficient, which can affect small intestine (mesenteric ischemia) or colon (ischemic colitis). The method of the present invention can be used for *in vitro* diagnosis of mesenteric ischemia-reperfusion, especially acute mesenteric ischemia or chronic mesenteric ischemia, ischemic colitis, or a disease or trouble linked to gut barrier. In a preferred embodiment, the method of the present invention is *for in vitro* diagnosing acute mesenteric ischemia.

According to the method of the present invention, the level of circulating glucagon-like peptide 1 or glucagon-like peptide 2 is determined by the mean value of an immunoassay.

An immunoassay is understood as different immunological techniques known from the one skilled in the art such as ELISA (Enzyme-Linked Immunosorbent Assay), Western-blot, RIA (radioimmunoassay), competitive EIA (competitive enzyme immunoassay), immunocytochemistry and immunohistochemistry techniques.

In an embodiment, the present invention concerns an *in vitro* diagnostic method for diagnosing and treating at an early stage an intestinal ischemia in a patient, which comprises:
(i) determining in a biological sample from said patient the circulating glucagon-like peptide 1 (GLP-1) level,
(ii) comparing said level with the circulating glucagon-like peptide 1 level in a reference sample,
(iii) applying a treatment of intestinal ischemia if the level of GLP-1 in said patient is increased at least 50%, 60%, 70%, 80%, 90%, 100%, 150%, 200%, 250%, or 300% higher than GLP-1 level measured at the same time in the reference sample.

Another embodiment of the present invention concerns an *in vitro* diagnostic method for diagnosing and treating at an early stage an intestinal ischemia in a patient, which comprises:
(i) determining in a biological sample from said patient the circulating glucagon-like peptide 2 level,
(ii) comparing said level with the circulating glucagon-like peptide 2 level in a reference sample,
(iii) applying a treatment of intestinal ischemia if the level of GLP-2 in said patient is increased at least 50%, 60%, 70%, 80%, 90%, 100%, 150%, 200%, 250%, or 300% higher than GLP-2 level measured at the same time in the reference sample.

Said intestinal ischemia treatment can be any treatment method known in prior art and applied in hospital.

In a particular embodiment, the immunoassay is an ELISA test, which uses at least one antibody with specificity for GLP-1 or an antibody with specificity for GLP-2. Said antibody can be a commercially available monoclonal or polyclonal antibody directed against GLP-1 or GLP-2.

The ELISA test can be carried out by a commercially available GLP-1 ELISA test or a commercially available GLP-2 ELISA test.

Said ELISA assay can be direct ELISA, indirect ELISA, sandwich ELISA, or competitive ELISA.

In a direct ELISA test, the presence of GLP-1 or of GLP-2 is directly indicated by a specific antibody conjugated with an enzyme; while in an indirect ELISA test, GLP-1 or GLP-2 is recognized by a first antibody specific to GLP-1 or GLP-2 protein, said first antibody being recognized by a second antibody conjugated with an enzyme.

The sandwich ELISA quantifies GLP-1 or GLP-2 protein between two layers of antibodies (i.e. capture and detection antibody). Either monoclonal or polyclonal antibodies can be used as the capture and detection antibodies in Sandwich ELISA systems.

Another aspect of the present invention concerns the use of a reagent capable of detecting the level of circulating glucagon-like peptide 1 or of circulating glucagon-like peptide 2 for diagnosing *in vitro* at an early stage an intestinal ischemia in a patient suspected of suffering from intestinal ischemia.

In a preferred embodiment, said reagent is an antibody directed against glucagon-like peptide 1 or glucagon-like peptide 2. Said antibody can be any conventional or commercially available monoclonal or polyclonal antibody known in prior art.

Particularly, the present invention concerns the use of a kit of ELISA containing an antibody directed against glucagon-like peptide 1 or glucagon-like peptide 2 for diagnosing *in vitro* at an early stage an intestinal ischemia in a patient suspected of suffering from intestinal ischemia.

The ELISA test can be carried out by a commercially available GLP-1 ELISA test or GLP-2 ELISA test.

The present invention is explained more in detail by following figures and examples.

### Figures

Figures 1A, 1B and 1C show intestinal injury following acute mesenteric ischemia and reperfusion. Hematoxylin / eosin staining of ileum histological sections after sham treatment (figure 1A), 20 minutes of ischemia followed by 15 minutes of reperfusion (figure 1B) and 40 minutes of ischemia followed by 15 minutes of reperfusion (figure 1C).

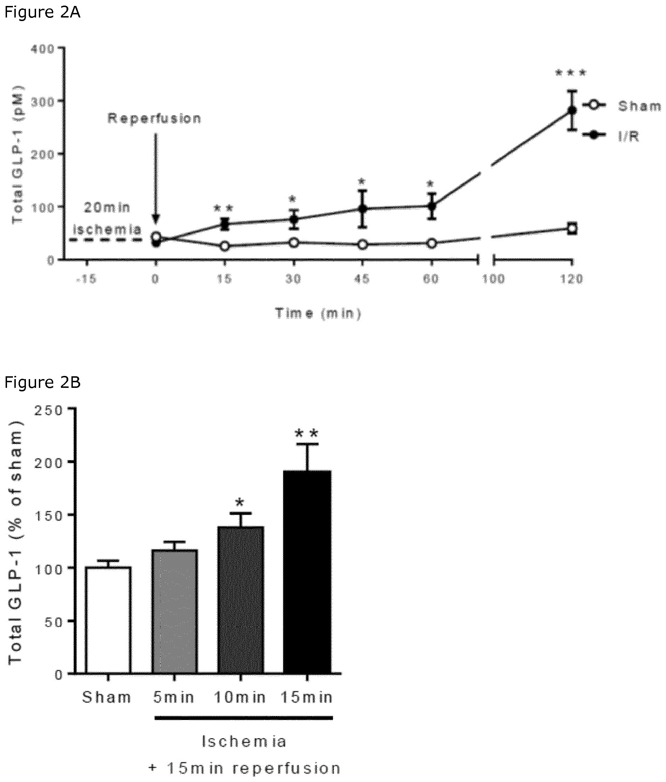

Figures 2A and 2B show that intestinal ischemia/reperfusion induces a quick secretion of GLP-1. Ischemia-reperfusion (I/R) of the superior mesenteric artery is applied in a group of 6 mice. Sham-operated mice were used as control. Statistical analysis were performed using an unpaired t test, difference from sham (*) are displayed: *p<0.05, **p<0.01 and ***p<0.001. Values are mean ± SEM. Figure 2A: total GLP-1 plasma levels (pM; n=5) after 20 minutes of ischemia followed by 15, 30, 45, 60 and 120 minutes of reperfusion. Figure 2B: total GLP-1 plasma levels (% of sham; n=5) after 5, 10 and 15 minutes of ischemia followed by 15min of reperfusion.
Figure 3: total GLP-1 and I-FABP plasma levels (% of sham, n=6) after a short and a long I/R (20 minutes ischemia followed by 15 minutes or 2 hours reperfusion respectively).
Figure 4 shows the comparison of the secretion of inflammation markers after a treatment of I/R (20min/30min) in mice of Sham group and of I/R group.
Figure 5 shows total GLP-1 arteriovenous differences in patients before and after 45 minutes ischemia and 0, 30 or 120 minutes reperfusion (n=6). All results are expressed as mean ± SEM.

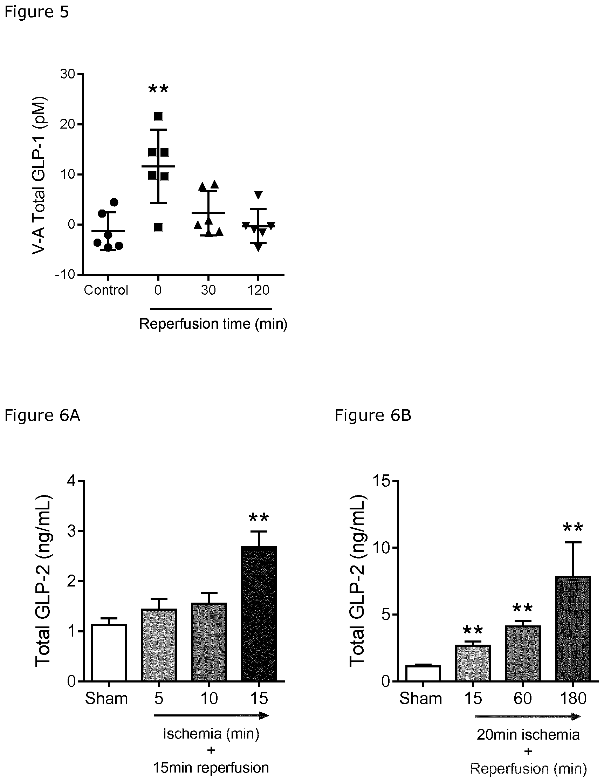

Figure 6A and 6B show that intestinal ischemia/reperfusion induces a quick secretion of total GLP-2. Ischemia-reperfusion (I/R) of the superior mesenteric artery is applied in a group of 6 mice. Sham-operated mice were used as control. Statistical analysis were performed using an unpaired t test, difference from sham (*) are displayed: *p<0.05, **p<0.01 and ***p<0.001. Values are mean ± SEM. Figure 6A: total GLP-2 plasma levels (ng/mL; n=5) after 5, 10 and 15 minutes of ischemia followed by 15min of reperfusion. Figure 6B: total GLP-2 plasma levels (ng/mL; n=5) after 20 minutes of ischemia followed by 15, 60 and 180 minutes of reperfusion.

### Examples

### 1. Materials and methods

### Animals

WT mice (8-12 weeks old, Charles River) from a homogeneous C57BL6/J background were housed in a controlled environment and fed a standard chow diet (A03 diet; Safe, Augy, France). Animals had free access to water and food. All experiments involving animals were performed in accordance with institutional guidelines and approved by the University of Burgundy's Ethics Committee on the Use of Laboratory Animals (protocol number 5459).

### Animal model of intestinal ischemia/reperfusion

Mice were separated into sham-operated groups and ischemia/reperfusion (I/R) groups (n=5/6). They were anesthetized with isoflurane inhalation and placed in a supine position on heating pads to maintain body temperature at 37°C. Midline laparotomy was performed and the superior mesenteric artery (SMA) was isolated. Ischemia was induced by clamping the SMA for 5, 10, 15 or 20 minutes and was followed by 15, 30, 45, 60 or 120 minutes of reperfusion (removal of the clamp). Gut ischemia was confirmed by intestinal color, change and gut reperfusion by the reappearance of pulsation and color. Blood collections were performed to quantify GLP-1, GLP-2 and cytokines. Blood samples were collected in EDTA-coated tubes (BD Vacutainer®) from the systemic (retro-orbital or intracardiac puncture) circulation. Plasma was separated by centrifugation at 8000 rpm for 10 minutes at 4°C. Blood and plasma samples were frozen at -20°C for further analysis. Mice were euthanatized by cervical dislocation and the distal part of the small intestine (ileum) was removed and immediately fixed for histological studies.

Surgical operation for sham-operated group mice was the same, except that the superior mesenteric artery was not clamped.

### Human intestinal ischemia/reperfusion

The experimental protocol was performed as previously described (Grootjans *et al.,* 2010). The study was approved by the Medical Ethics Committee of the Maastricht University Medical Center and written informed consent of all patients was obtained. 6 patients with a median age of 66 years (range, 54 to 83 years) undergoing pancreaticoduodenectomy for benign or malignant disease were included in this study. Patients with bile duct obstructive disease were stented before surgery. All patients had normal bile flow at the time of the surgical procedure. During pancreaticoduodenectomy, a variable segment of jejunum is routinely resected in continuity with the head of the pancreas and duodenum as part of the surgical procedure. The terminal 6 cm of this jejunal segment was isolated and subjected to 45 minutes of ischemia by placing two atraumatic vascular clamps over the mesentery. Meanwhile, surgery proceeded as planned. After 45 minutes of ischemia, one third (2cm) of the isolated ischemic jejunum was resected using a linear cutting stapler. Next, clamps were removed to allow reperfusion, as confirmed by regaining of normal pink color and restoration of gut motility. Another segment of the isolated jejunum (2cm) was resected similarly after 30 minutes of reperfusion. The last part was resected after 120 minutes of reperfusion. Simultaneously, 2 cm of jejunum, which remained untreated during surgery, was resected, serving as internal control tissue. This segment underwent similar surgical handling as the isolated part of jejunum, but was not exposed to I/R. Arterial blood was sampled before ischemia, immediately on reperfusion, and at 30 and 120 minutes after start of reperfusion. Simultaneous with each respective arterial blood sample, blood was drawn from the venule draining the isolated jejunal segment by direct puncture to assess concentration gradients across the isolated jejunal segment. All blood samples were directly transferred to prechilled EDTA vacuum tubes (Becton Dickinson Diagnostics, Aalst, Belgium) and kept on ice. At the end of the procedure all blood samples were centrifuged at 4000 rpm, 4 °C for 15 minutes to obtain plasma. Plasma was immediately stored in aliquots at -80°C until analysis.

### Light microscopy

The morphologic alterations in the gut were examined by light microscopy (x50, x100 and x200). Briefly, tissues from the distal small intestine (ileum) were promptly taken in sham-operated and I/R groups after 20 or 40 minutes of ischemia and 15 minutes of reperfusion. Gut samples were fixed for 48 hours in 10% neutral buffered formalin at room temperature, dehydrated by graded ethanol and embedded in paraffin for histological analysis. Tissue sections (thickness of 5µm) were deparaffinized with xylene, stained with hematoxylin and eosin.

### Biochemical Analysis

Total GLP-1, GLP-2 and I-FABP concentrations were determined by commercially available ELISA Kits (Millipore, St. Charles, MO and Cliniscience) in accordance with manufacturer's protocols.

Cytokines plasma levels (interleukin (IL)-1β, IL-6 and tumor necrosis factor-a (TNF-a)) were measured by Milliplex MAP 5-Plex Kit using mouse cytokine/chemokine magnetic bead panel (Millipore, Billerica, MA) according to the manufacturer's protocol and using a LuminexR apparatus (Bio-Plex 200, Bio-Rad).

### Statistical Analysis

Numeric data are presented as mean ± standard error of mean. Statistical analysis were performed using either the unpaired Sudent's t-test or the nonparametric Mann-Withney U test depending on data distribution's normality. D'Agostino's K² test was used to establish whether or not groups of data were normally distributed. A statistical correction was applied when variances were different between groups. A value of P < 0.05 was considered statistically significant.

### 2. Results

### 2.1. Rapid GLP-1 secretion in mice after gut barrier injury

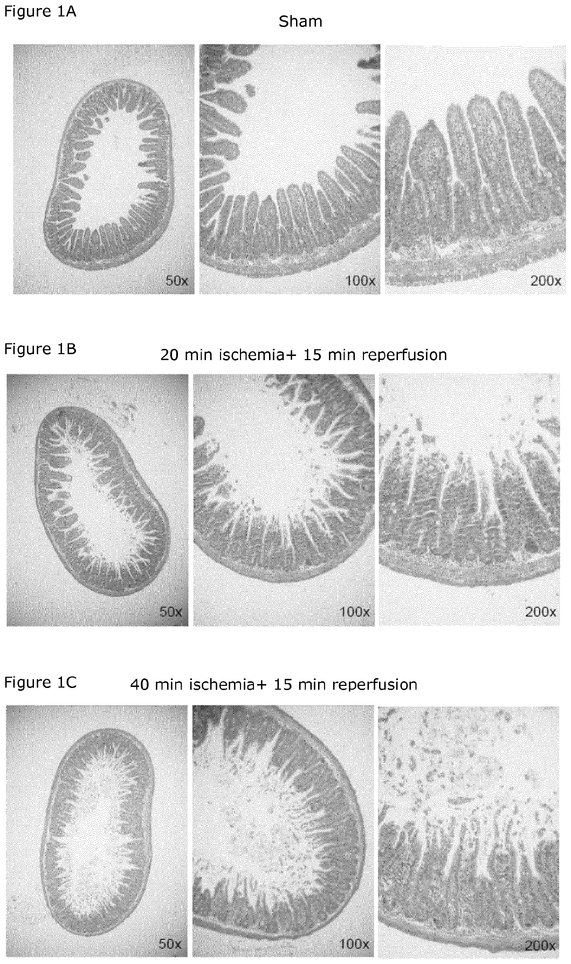

The mesenteric ischemia-reperfusion (I/R) was produced in a group of mice. A group of sham-operated mice was used as control. Gut ultrastructure was deeply disorganized after I/R (Figures 1A, 1B, 1C). Short times of I/R were sufficient to damage intestinal villi compared to sham-operated mice (Figure 1A and 1B). Increased damages were observed with increased times of I/R (Fig. 1C). I/R experiments led to a rapid increase of GLP-1 plasma levels (Figures 2A and 2B). As shown in Fig. 2A, reperfusion of the mesenteric artery for 2 hours after 20 minutes of ischemia led to a raise of GLP-1 plasma levels. More notably, this increase was significant after only 15 minutes of reperfusion. Shorter times of ischemia (< 20 minutes) were still able to induce GLP-1 secretion (Fig 2B).

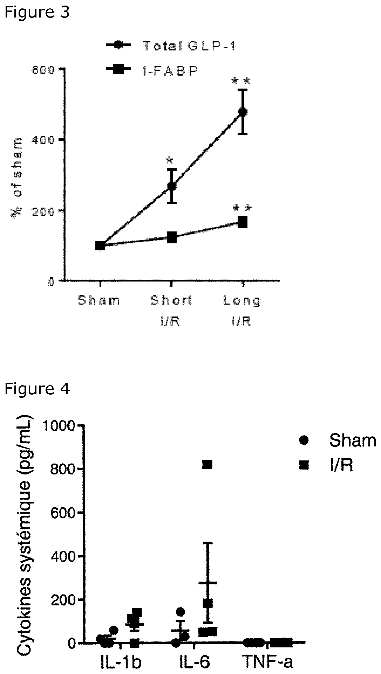

It is revealed that after mesenteric ischemia-reperfusion, GLP-1 secretion precedes I-FABP secretion (Figure 3) and markers of inflammation, such as IL-1b, IL-6, TNF-a (Figure 4). In contrast to I-FABP, a short I/R treatment was sufficient to induce a significant plasma GLP-1. Moreover, after mesenteric ischemia-reperfusion, GLP-1 secretion is quantitatively more important than that of I-FABP (Figure 3).

These results show that, compared with I-FABP, GLP-1 is more sensitive and can be used as biomarker for diagnosing gut barrier injury at more early stage.

### 2.2. Rapid GLP-1 secretion in human after gut barrier injury

A new model of I/R using human gut tissue (Grootjans et al., 2010) has been used to evaluate if I/R injury in the human gut *in vivo* was associated with an increase in GLP-1 secretion. Arterio-venous differences in human plasma GLP-1 levels were measured before, after 45 minutes of ischemia and after 30 or 120 minutes of reperfusion. GLP-1 levels were markedly increased after 45 minutes of ischemia and returned to baseline levels after reperfusion (Figure 5). These results demonstrate that human gut injury is associated with a rapid induction of GLP-1 secretion *in vivo.*

### 2.3. Rapid GLP-2 secretion in mice after gut barrier injury

GLP-2 secretion level is also measured in mice after gut barrier injury in the experimental conditions similar to that for measuring GLP-1 secretion level. A rapid increase of plasma GLP-2 level is observed after a very short time (15 minutes) of intestinal ischemia (Figure 6A). An important increase of plasma GLP-2 level is observed after 180 minutes of reperfusion following 20 minutes of ischemia (Figure 6B).

These results shows that GLP-2 is also sensitive to gut barrier injury and can be used as a biomarker for diagnosing gut barrier injury at more early stage.

## Claims

1. An *in vitro* diagnostic method for diagnosing at an early stage an intestinal ischemia in a patient suspected of suffering from intestinal ischemia, which comprises:
(i) determining in a biological sample from said patient the circulating glucagon-like peptide 2 (GLP-2) level and/or the circulating glucagon-like peptide 1 (GLP-1) level,
(ii) comparing said level with the circulating glucagon-like peptide 2 level and/or the circulating glucagon-like peptide 1 level in a reference sample,
wherein the increase of said level in the patient is indicative of said patient suffering from intestinal ischemia.

2. The method according to claim 1, wherein the intestinal ischemia is mesenteric ischemia-reperfusion, especially acute mesenteric ischemia, chronic mesenteric ischemia, or ischemic colitis, or a disease or trouble linked to gut barrier.

3. The method according to claim 1 or 2, wherein the biological sample is a blood plasma sample.

4. The method according to any one of claims 1 to 3, wherein the level of circulating glucagon-like peptide 2 or circulating glucagon-like peptide 1 is determined by the means of an immunoassay.

5. The method according to claim 4, wherein said immunoassay is ELISA.

6. The method according to any one of claims 1 to 5, wherein the reference sample is a biological sample from a healthy subject.

7. Use of a reagent capable of detecting the level of circulating glucagon-like peptide 2 or of circulating glucagon-like peptide 1 for diagnosing *in vitro* at an early stage an intestinal ischemia in a patient suspected of suffering from intestinal ischemia.

8. The use according to claim 7, wherein the reagent is an antibody directed against glucagon-like peptide 2 or glucagon-like peptide 1.

9. Use of a kit of ELISA containing an antibody directed against glucagon-like peptide 2 or glucagon-like peptide 1 for diagnosing *in vitro* at an early stage an intestinal ischemia in a patient suspected of suffering from intestinal ischemia.

## Patentansprüche

1. In-vitro-Diagnoseverfahren zur Diagnose von Darmischämie im Frühstadium bei einem Patienten mit Verdacht auf Darmischämie, welches umfasst:
(i) Bestimmen des Niveaus von zirkulierendem Glucagon-like Peptide 2 (GLP-2) und/oder des Niveaus von zirkulierendem Glucagon-like Peptide 1 (GLP-1) in einer biologischen Probe von dem Patienten;
(ii) Vergleichen des Niveaus mit dem Niveau des zirkulierenden Glucagon-like Peptide 2 und/oder dem Niveau des zirkulierenden Glucagon-like Peptide 1 in einer Bezugsprobe,
wobei der Anstieg des Niveaus bei dem Patienten darauf hinweist, dass der Patient an einer Darmischämie leidet.

2. Verfahren nach Anspruch 1, wobei die Darmischämie eine Mesenterialischämie-Reperfusion ist, insbesondere eine akute Mesenterialischämie, eine chronische Mesenterialischämie oder eine ischämische Kolitis, oder eine mit einer Darmbarriere verbundene Krankheit oder Beschwerde.

3. Verfahren nach Anspruch 1 oder 2, wobei die biologische Probe eine Blutplasmaprobe ist.

4. Verfahren nach einem der Ansprüche 1 bis 3, wobei das Niveau an zirkulierendem Glucagon-like Peptide 2 oder zirkulierendem Glucagon-like Peptide 1 mittels eines Immunoassays bestimmt wird.

5. Verfahren nach Anspruch 4, wobei der Immunoassay ein ELISA ist.

6. Verfahren nach einem der Ansprüche 1 bis 5, wobei die Bezugsprobe eine biologische Probe von einer gesunden Person ist.

7. Verwendung eines Reagenzes zum Nachweis des Niveaus von zirkulierendem Glucagon-like Peptide 2 oder von zirkulierendem Glucagon-like Peptide 1 zur in-vitro-Diagnose einer Darmischämie im Frühstadium bei einem Patienten, bei dem Verdacht auf Darmischämie besteht.

8. Verwendung nach Anspruch 7, wobei das Reagenz ein Antikörper ist, der gegen das Glucagon-like Peptide 2 oder das Glucagon-like Peptide 1 gerichtet ist.

9. Verwendung eines ELISA-Kits, das einen gegen Glucagon-like Peptide 2 oder Glucagon-like Peptide 1 gerichteten Antikörper enthält, zur in-vitro-Diagnose einer Darmischämie im Frühstadium bei einem Patienten, bei dem Verdacht auf Darmischämie besteht.

## Revendications

1. Une méthode de diagnostic *in vitro* pour diagnostiquer à un stade précoce une ischémie intestinale chez un patient suspecté de souffrir d'une ischémie intestinale, qui comprend :
(i) la détermination dans un échantillon biologique dudit patient du taux de glucagon-like peptide 2 circulant (GLP-2) et/ou du taux de glucagon-like peptide 1circulant (GLP-1),
(ii) la comparaison dudit taux avec le taux de glucagon-like peptide 2 circulant et/ou de glucagon-like peptide 1 circulant dans un échantillon de référence,
dans laquelle l'augmentation dudit taux chez le patient est indicatif du fait que ce patient souffre d'une ischémie intestinale.

2. La méthode selon la revendication 1, dans laquelle l'ischémie intestinale est une ischémie de reperfusion mésentérique, en particulier une ischémie mésentérique aigue, une ischémie mésentérique chronique ou une colite ischémique ou une maladie ou un trouble lié à la barrière intestinale.

3. La méthode selon la revendication 1 ou 2, dans lequel l'échantillon biologique est un échantillon de plasma sanguin.

4. La méthode selon l'une quelconque des revendications 1 à 3, dans laquelle le taux de glucagon-like peptide 2 circulant ou de glucagon-like peptide 1 circulant est déterminé au moyen d'un immunoessai.

5. La méthode selon la revendication 4, dans laquelle l'immunoessai est un ELISA.

6. La méthode selon l'une quelconque des revendications 1 à 5, dans laquelle l'échantillon de référence est un échantillon biologique provenant d'un sujet sain.

7. Utilisation d'un réactif capable de détecter le taux de glucagon-like peptide 2 circulant ou de glucagon-like peptide 1 circulant pour diagnostiquer *in vitro* à un stade précoce une ischémie intestinale chez un patient suspecté de souffrir d'une ischémie intestinale.

8. Utilisation selon la revendication 7, dans laquelle le réactif est un anticorps dirigé contre le glucagon-like peptide 2 ou le glucagon-like peptide 1.

9. Utilisation d'un kit ELISA contenant un anticorps dirigé contre le glucagon-like peptide 2 ou le glucagon-like peptide 1 pour diagnostiquer *in vitro* à un stade précoce une ischémie intestinale chez un patient suspecté de souffrir d'une ischémie intestinale.
